# EUROPEAN PATENT APPLICATION

(11) **EP 2 889 040 A1**
(43) Date of publication of application: **01.07.2015**
(21) Application number: 13832769.7
(22) Date of filing: 21.08.2013
(51) Int. Cl.: A61K 39/002, A61K 9/127, A61K 47/26, A61K 47/36, A61P 33/02, A61P 37/04, C07K 14/45

(54) **VACCINE FORMULATION AGAINST TOXOPLASMA GONDII INFECTION**

(30) Priority: 27.08.2012 JP 2012186205
(71) Applicant: Obihiro University Of Agriculture And Veterinary Medicine, Obihiro-shi, Hokkaido 080-8555 (JP)
(72) Inventor: NISHIKAWA, Yoshifumi, Obihiro-shi Hokkaido 080-8555 (JP); KURODA, Yasuhiro, Hiratsuka-shi Kanagawa 259-1292 (JP); KOJIMA, Naoya, Hiratsuka-shi Kanagawa 259-1292 (JP)
(74) Representative: Hoffmann Eitle
(86) International application number: PCT/JP2013/004928
(87) International publication number: WO 2014/034046

(57) **Abstract**

The present invention relates to a vaccine formulation against *Toxoplasma gondii* infection. More specifically, the invention relates to a vaccine formulation in which a *Toxoplasma gondii*-derived soluble protein is included in liposomes each having an oligosaccharide capable of binding to a carbohydrate recognition molecule on the surface of antigen-presenting cells on the surface of the liposome.

SEQUENCE LISTING

<110>  Obihiro University of Agriculture and Veterinary Medicine
       Tokai University Educational System

<120>  Vaccine preparation against toxoplasma infection

<130>  POC-9002WO

<150> JP2012-186205
<151> 2012-08-27

<160>  2

<170>  PatentIn version 3.1

<210>  1
<211>  492
<212>  DNA
<213>  Toxoplasma gondii

<220>
<221>  CDS
<222>  (1)..(492)
<223>

<400>  1
atg tcc gac tgg gac cct gtt gtc aag gag tgg ctt gtt gac aca ggc       48
Met Ser Asp Trp Asp Pro Val Val Lys Glu Trp Leu Val Asp Thr Gly
1               5                   10                  15

tac tgc tgc gca ggc ggc atc gcc aac gcg gag gac ggt gtt gtg ttc       96
Tyr Cys Cys Ala Gly Gly Ile Ala Asn Ala Glu Asp Gly Val Val Phe
            20                  25                  30

gcc gcg gcg gct gat gat gat gac gga tgg tcc aag ctg tac aag gat      144
Ala Ala Ala Ala Asp Asp Asp Asp Gly Trp Ser Lys Leu Tyr Lys Asp
        35                  40                  45

gat cat gag gag gac act atc gga gag gat ggc aac gcg tgc ggc aag      192
Asp His Glu Glu Asp Thr Ile Gly Glu Asp Gly Asn Ala Cys Gly Lys
    50                  55                  60

gtg tcg atc aac gag gcc tcc acg atc aaa gct gca gtt gac gat ggc      240
Val Ser Ile Asn Glu Ala Ser Thr Ile Lys Ala Ala Val Asp Asp Gly
65                  70                  75                  80

agt gcc cct aac ggt gtt tgg att ggc ggc cag aag tac aag gtt gtc      288
Ser Ala Pro Asn Gly Val Trp Ile Gly Gly Gln Lys Tyr Lys Val Val
                85                  90                  95

cga cct gag aaa gga ttc gag tac aac gac tgc acc ttc gac atc acc      336
Arg Pro Glu Lys Gly Phe Glu Tyr Asn Asp Cys Thr Phe Asp Ile Thr
            100                 105                 110

atg tgt gca cgg tcc aag ggt ggc gcg cac ttg atc aag acc ccg aat      384
Met Cys Ala Arg Ser Lys Gly Gly Ala His Leu Ile Lys Thr Pro Asn
        115                 120                 125

ggc tct atc gtc att gcc ctt tac gat gag gag aag gaa cag gac aag      432
Gly Ser Ile Val Ile Ala Leu Tyr Asp Glu Glu Lys Glu Gln Asp Lys
    130                 135                 140

gga aac agc agg act tcg gca ttg gcc ttt gcc gag tat ctt cac cag 480
Gly Asn Ser Arg Thr Ser Ala Leu Ala Phe Ala Glu Tyr Leu His Gln
145                 150                 155                 160

tct ggg tac taa 492
Ser Gly Tyr

<210> 2
<211> 163
<212> PRT
<213> Toxoplasma gondii

<400> 2

Met Ser Asp Trp Asp Pro Val Val Lys Glu Trp Leu Val Asp Thr Gly
1               5                   10                  15

Tyr Cys Cys Ala Gly Gly Ile Ala Asn Ala Glu Asp Gly Val Val Phe
            20                  25                  30

Ala Ala Ala Ala Asp Asp Asp Asp Gly Trp Ser Lys Leu Tyr Lys Asp
        35                  40                  45

Asp His Glu Glu Asp Thr Ile Gly Glu Asp Gly Asn Ala Cys Gly Lys
    50                  55                  60

Val Ser Ile Asn Glu Ala Ser Thr Ile Lys Ala Ala Val Asp Asp Gly
65                  70                  75                  80

Ser Ala Pro Asn Gly Val Trp Ile Gly Gly Gln Lys Tyr Lys Val Val
                85                  90                  95

Arg Pro Glu Lys Gly Phe Glu Tyr Asn Asp Cys Thr Phe Asp Ile Thr
            100                 105                 110

Met Cys Ala Arg Ser Lys Gly Gly Ala His Leu Ile Lys Thr Pro Asn
        115                 120                 125

Gly Ser Ile Val Ile Ala Leu Tyr Asp Glu Glu Lys Glu Gln Asp Lys
    130                 135                 140

Gly Asn Ser Arg Thr Ser Ala Leu Ala Phe Ala Glu Tyr Leu His Gln
145                 150                 155                 160

Ser Gly Tyr

1

## Description

### Technical Field

The present invention relates to a vaccine formulation against *Toxoplasma gondii* infection and more specifically to a vaccine formulation in which a *Toxoplasma gondii*-derived soluble protein is included in liposomes each having an oligosaccharide capable of binding to a carbohydrate recognition molecule on the surface of antigen-presenting cells on the surface of the liposome.

### Background Art

*Toxoplasma gondii* is an intracellular protozoan parasite which can infect to an animal belonging to the cat family as a final host and a human, a pig, sheep, a goat, a bird, or the like as an intermediate host. *Toxoplasma gondii* is transmitted by horizontal infection from oocyst discharged in the feces of a final host or by vertical infection in an intermediate host. Toxoplasmosis runs its course as a subclinical infection in the case where the host's immunocompetence is normal; however, in humans, it is an opportunistic infection occurring when the host falls into the state of immunosuppression due to AIDS, organ transplantation, pregnancy, or the like. In animals, *Toxoplasma gondii* infection for which abortion and stillbirth due to the infection in pigs and sheep are industrially problematical is identified all over the world, and infection routes include direct infection to a human and infection through the meat of an infected animal.

The following needs to be considered for a vaccine for preventing the *Toxoplasma gondii* infection. Because *Toxoplasma gondii* is an intracellular protozoan parasite, cellular immunity is important for protective immunity thereagainst. Particularly, the function of interferon gamma and CD8-positive T cells is important. The effect of antibody on *Toxoplasma gondii* is a controversial subject; however, the antibody is believed to react to *Toxoplasma gondii* present outside the cells to prevent the spread thereof in the body.

Infection during pregnancy causes miscarriage or vertical infection due to the characteristic immune response of the mother's body. In other words, infection with *Toxoplasma gondii* during early pregnancy induces abortion due to the inflammatory reaction caused in the mother's body. When *Toxoplasma gondii* infection occurs during mid-pregnancy during which the mother's body is immunosuppressed, vertical infection to the fetus is established, and an individual congenitally infected with *Toxoplasma gondii* is born.

Some vaccines against *Toxoplasma gondii* using inactivated and live forms of the protozoon have previously been attempted to be developed on animal toxoplasmatosis. The vaccine strain S48 of *Toxoplasma gondii* is known as a strain having reduced pathogenicity, and used in a currently marketed live vaccine (Toxovax (R)). In New Zealand, the preventive effect of the S48 vaccine has been confirmed against sheep abortion caused by infection with *Toxoplasma gondii* (Non Patent Literatures 1 and 2). Problems of the vaccine include necessary transportation under cold storage and a short expiration date, and possible reversion of pathogenicity. No successful example in domestic animals is reported for the inactivated vaccine of *Toxoplasma gondii.* A vaccine in which a protozoal antigen is included in immunostimulating complexes (ISCOMS) has not been shown to have a vaccine effect in sheep and pigs (Non Patent Literatures 3 and 4). A DNA vaccine encoding a protozoal antigen as a subunit vaccine is reported to induce specific immunity in pigs, but has not been shown to have a protective effect against protozoal infection (Non Patent Literature 5).

To control *Toxoplasma gondii* infection, it is necessary to induce parasite-specific cellular immunity in host animals. The live vaccine can be expected to induce the cellular immunity; however, there is concern that the live vaccine has its own pathogenicity, having the risk of expanding the infection. The inactivated vaccine and the subunit vaccine are highly safe, but have a disadvantage of not sufficiently inducing an immune response. Thus, to develop a more effective vaccine, it is important to discover a new combination of a safe recombinant antigen capable of effectively inducing T cell immunity against the parasite and an adjuvant medium.

Liposomes coated with a macromolecular polysaccharide such as mannan developed as an adjuvant for vaccines and immunotherapy are reported to have a strong ability to induce cellular immunity (Patent Literature 1 and Non Patent Literature 6). However, mannan is a mixture of polymannoses with heterogeneous molecular weights and also has strong toxicity to a living body (Non Patent Literature 7); thus, it is not suitable for a pharmaceutical product.

Meanwhile, Mizuochi et al. reports that an antigen is included in liposomes whose surface has an oligosaccharide comprising 2 to 11 sugar residues and binding to a lectin derived from antigen-presenting cells to remove the toxicity and antigenicity of the sugar and enhance its effect as a vaccine (Patent Literature 2). This document also discloses that cellular immunity against the antigen included in the liposomes each having the oligosaccharide on the surface can be efficiently induced. The liposome having the oligosaccharide on the surface is thought to be phagocytized by antigen-presenting cells via mannose receptors, resulting in the presentation of the antigen through an MHC class I or II molecule for the activation of antigen-specific T cells and the induction of Th1-derived cytokines.

Shimizu et al. report that when a *Leishmania major-*derived soluble antigen is included in liposomes each having an oligosaccharide on the surface and mice are immunized with these liposomes, Th1-type immunity against *Leishmania major* is significantly induced to control the infection of the mice with the protozoon (Non Patent Literature 8).

Yokoyama and Kuboki et al. report that a *Toxoplasma gondii-*derived soluble antigen included in oligomannose carbohydrate-coated liposomes can induce an antigen-specific Th1-type immune response (Non Patent Literatures 9 and 10) and that the oligomannose carbohydrate-coated liposome has an adjuvant effect (Non Patent Literature 11). However, the material here used as the antigen is a soluble component of *Toxoplasma gondii* cells, containing various antigen peptides.

Nishikawa et al. also report that the inclusion of an *Neospora caninum-*derived soluble antigen in liposomes having an oligosaccharide on the surface and the immunization of mice with the liposomes significantly induce Th1-type immunity against *Neospora caninum* and control the vertical infection of the parasite and the spread thereof in the body (Patent Literature 3 and Non Patent Literature 12).

### Citation List

### Patent Literature

Patent Literature 1: International Publication No. WO 92/04887
Patent Literature 2: Japanese Patent Laid-Open No. 7-126185 (Japanese Patent No. 2828391)
Patent Literature 3: International Publication No. WO 2010/32408

### Non Patent Literature

Non Patent Literature 1: O'Connell et al., N Z Vet J. 1988, 36:1-4.
Non Patent Literature 2: Wilkins et al., N Z Vet J. 1988, 36:86-89.
Non Patent Literature 3: Buxton et al., Br Vet J. 1989, 145:451-457.
Non Patent Literature 4: Garcia et al., Vet Parasitol. 2005, 129:209-217.
Non Patent Literature 5: Jongert et al., Vaccine. 2008, 26:1025-1031.
Non Patent Literature 6: Noguchi et al., J. Immunol. 1989, 143:3737-3742.
Non Patent Literature 7: Mikami et al., 15th Carbohydrate Symposium, Abstract 1993, 43-44.
Non Patent Literature 8: Shimizu et al., Parasite Immunol. 2007, 29: 229-239.
Non Patent Literature 9: Naoaki Yokoyama et al., Japanese Society of Parasitology Convention Program/Proceeding, 2006, p. 67, I-D-04
Non Patent Literature 10: Kuboki et al., J. Protozool Res., 2007, 17:9-15.
Non Patent Literature 11: Kuboki et al., J. Protozool Res., 2008, 18:1-10.
Non Patent Literature 12: Nishikawa et al., Clin Vaccine Immunol. 2009, 16:792-797.

### Summary of Invention

### Technical Problem

An object of the present invention is to provide a safe vaccine formulation capable of inducing an effective immune response to *Toxoplasma gondii* infection and to achieve the enhancement of a prophylactic effect against the *Toxoplasma gondii* infection.

### Solution to Problem

As a result of intensive studies for solving the above-described problems, the present inventors have found that only the use of profilin as an antigen among various *Toxoplasma gondii*-derived proteins achieves a high vaccine effect. Although a molecule selected as a vaccine antigen is typically a secretory antigen or a cell membrane antigen, profilin is known as a protein located in the cytoplasm and thus has been difficult to predict as a vaccine antigen.

The present invention is based on such findings and relates to a liposome formulation in which *Toxoplasma gondii*-derived profilin or an immunologically active variant or derivative thereof, or an immunologically active fragment thereof is included in liposomes each having an oligosaccharide capable of binding to a carbohydrate recognition molecule on the surface of antigen-presenting cells on the surface of the liposome.

Examples of the carbohydrate recognition molecule on the surface of antigen-presenting cells can include a mannose receptor.

The immunologically active fragment is not particularly limited; however, the fragment may be a fragment comprising at least continuous 8 to 50 amino acid residues of the amino acid residues of profilin or an immunologically active variant or derivative thereof.

In one aspect, profilin or an immunologically active variant or derivative thereof is any protein of the following (a) to (c).
(a) a protein comprising the amino acid sequence shown in SEQ ID NO: 2,
(b) a protein comprising an amino acid sequence in which one or several amino acids are deleted, substituted, or added in the amino acid sequence shown in SEQ ID NO: 2 and capable of inducing an immune response to *Toxoplasma gondii,* and
(c) a protein comprising an amino acid sequence having 80% or more identity with the amino acid sequence shown in SEQ ID NO: 2 and capable of inducing an immune response to *Toxoplasma gondii.*

The oligosaccharide used consists preferably of 2 to 11 sugar residues, more preferably of 3 to 7 sugar residues, most preferably of 3 to 5 sugar residues.

Preferred oligosaccharides can include a carbohydrate comprising 2 or more mannose.

The liposome formulation of the present invention can be used as a vaccine formulation for *Toxoplasma gondii* infection. It is desirable that the vaccine formulation be formulated together with a pharmaceutically acceptable carrier and administered, for example, subcutaneously, intradermally, intravenously, orally, ophthalmically, enterally, or nasally.

### Advantageous Effects of Invention

The liposome formulation provided in the present invention can induce antigen-specific Th1 immunity and is useful as a vaccine formulation against *Toxoplasma gondii* infection. The vaccine formulation of the present invention has a high prophylactic effect while having a reduced risk of side effects. Use of the vaccine formulation according to the present invention can prevent the vertical infection of fetuses with *Toxoplasma gondii* as well as the infection of vaccinated individuals therewith.

### Brief Description of Drawings

[Figure 1] Figure 1 is a graph showing the survival rate of mice during 21 days after infection.
[Figure 2] Figure 2 is a graph showing the survival rate of mice during 21 days after infection.
[Figure 3] Figure 3 is a graph showing the survival rate of mice during 30 days after infection.
[Figure 4] Figure 4 is a graph showing the production of a specific antibody after vaccination.
[Figure 5] Figure 5 is a pair of graphs showing cytokine production in spleen cells to various antigen stimulations.

The present specification encompasses the content of the specification of Japanese Patent Application No. 2012-186205 on which the priority of the present application is based.

### Description of Embodiments

The present invention relates to a liposome formulation in which *Toxoplasma gondii*-derived profilin or an immunologically active variant or derivative thereof is included in liposomes each having an oligosaccharide capable of binding to a carbohydrate recognition molecule on the surface of antigen-presenting cells on the surface of the liposome, and a vaccine formulation for *Toxoplasma gondii* infection comprising the liposome formulation.

The present invention will be described below in detail.

### 1. Toxoplasma gondii

A protozoon is a eukaryotic unicellular microorganism and is an animal unicellular organism having motor and predatory abilities. For differentiation from a unicellular parasite, a parasitic and particularly pathogenic organism is also often called a protozoon. Protozoa include a highly host-specific type capable of being parasitic only in humans (*Plasmodium spp., Isospora spp.,* or the like) and a type parasitic in a plurality of animal species and causing zoonotic infection (*Entamoeba histolytica, Cryptosporidium,* or the like). They vary in pathogenicity from those inducing lethal infections or serious symptoms to those inducing no symptom and having non-pathogenicity. Protozoa parasitic in the digestive tract orally invade a human body via drinking water or food. The infection form varies depending on the type of the protozoon; examples thereof include an encysted cyst, an oocyst, and a spore. *Entamoeba histolytica, Giardia lamblia,* and the like form cysts, *Coccidia* form oocysts, and *Microsporidia* form spores. Most protozoa parasitic in the blood and tissue proliferate in the intestinal tract of certain blood sucking insects and ixodids, and infect a human body using these as mediators.

*Toxoplasma gondii* is an intracellular parasitic protozoon utilizing an animal belonging to the feline family as a final host and a mammal, a bird, or the like as an intermediate host. *Toxoplasma gondii* is transmitted by horizontal infection from oocyst discharged in the feces of a final host or by vertical infection in an intermediate host. Toxoplasmosis runs its course as a subclinical infection in the case where the host's immunocompetence is normal; however, in humans, it is an opportunistic infection occurring when the host falls into the state of immunosuppression due to AIDS, organ transplantation, pregnancy, or the like. In animals, *Toxoplasma gondii* infection for which abortion and stillbirth due to the infection in sheep and pigs are industrially problematical is identified all over the world, and infection routes include direct infection to a human and infection through the meat of an infected animal. A sulfa drug, pyrimethamine, or spiramycin is used for the treatment thereof.

Some vaccines against *Toxoplasma gondii* using inactivated and live forms of the protozoon have previously been attempted to be developed on animal toxoplasmatosis. The vaccine strain S48 of *Toxoplasma gondii* is known as a strain having reduced pathogenicity, and used in a currently marketed live vaccine (Toxovax^{®}). In New Zealand, the preventive effect of the S48 vaccine has been confirmed against sheep abortion caused by protozoal infection; however, concerns exist about the reversion of pathogenicity of the vaccine strain. No successful example in domestic animals is reported for the inactivated vaccine of *Toxoplasma gondii.* The vaccine in which a protozoal antigen is included in immunostimulating complexes (ISCOMS) has not been shown to have a vaccine effect in sheep and pigs. The DNA vaccine encoding a protozoal antigen as a subunit vaccine is reported to induce specific immunity in pigs, but has not been shown to have a protective effect against protozoal infection.

As described above, research on the liposome vaccine is also reported in which a soluble antigen of *Toxoplasma gondii* is included in oligomannose carbohydrate-coated liposomes; however, the soluble antigen used here comprises various antigen proteins and cannot be said to have sufficient uniformity and safety as a vaccine formulation. Thus, at present, no safe and effective vaccine is developed against *Toxoplasma gondii* infection.

### 2. Profilin

According to the present invention, profilin is used as *Toxoplasma gondii*-derived soluble proteins for inducing immune response.

Profilin is an antigen molecule present in the cytoplasm in *Toxoplasma gondii.* Profilin is known to act on the Toll-like receptor 11 (TLR11) of mice and induce the production of interleukin 12 (IL-12) in dendritic cells. The amino acid sequence of profilin and the gene sequence encoding the same are already known and deposited in the public database GenBank under Accession Number AY897579 (*Toxoplasma gondii* inflammatory profilin mRNA, complete cds).

As for profilin used for the present invention, when an amino sequence from the initiation codon to the stop codon is used, the length of the amino acid sequence is not limited, and the profilin may be an immunologically active fragment.

The amino acid sequence of *Toxoplasma gondii*-derived profilin is shown in SEQ ID NO: 2. However, the amino acid sequence of *Toxoplasma gondii*-derived profilin is not limited to the amino acid sequence shown in SEQ ID NO: 2, and may be a sequence in which one or several amino acids are deleted, substituted, or added in the amino acid sequence shown in SEQ ID NO: 2, provided that it has a desired immunogenicity. The term "several amino acids" preferably means 2 to 7, more preferably 2 to 5, most preferably 2 to 3 amino acids. The amino acid substitution is preferably conservative substitution between analogous amino acid residues; examples thereof can include substitution between amino acids such as glycine (Gly) and proline (Pro), glycine and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), threonine and serine (Ser) or alanine, and lysine (Lys) and arginine (Arg).

Alternatively, the amino acid sequence may be a protein having a homology (identity) of at least about 70% or more, preferably about 80% or more, more preferably about 90% or more, still more preferably about 95% or more, particularly preferably about 97%, about 98% or about 99% or more with the amino acid sequence shown in SEQ ID NO: 2 when calculated using BLAST or the like (for example, when the default in Blast, that is, the initial condition parameters, are used), provided that it has a desired immunogenicity.

Thus, the "immunologically active variant or derivative" according to the present invention includes a slightly altered or modified profilin protein, a fused protein between profilin and another peptide, or the like as described above, provided that it can induce immune response activity against *Toxoplasma gondii* in a living body to which it was administered (for example, antigenicity, receptor binding, formation of a complex by the binding of the peptide by an MHC class I or class II molecule, and the like).

For the purpose of the present invention, the "immunologically active fragment" is not particularly limited; however, it means a fragment comprising at least continuous 8 to 50 amino acid residues of the amino acid residues of the above-described profilin or immunologically active variant or derivative thereof and capable of inducing an immune response activity against *Toxoplasma gondii.*

### 3. Preparation of profilin

### 3.1 Preparation from Toxoplasma gondii:

The preparation of profilin used for the present invention can be by a method which involves performing purification by general column work from a natural product containing a *Toxoplasma gondii*-derived soluble protein, e.g., *Toxoplasma gondii;* the step of subjecting the resultant *Toxoplasma gondii-derived* soluble protein to partial decomposition for carbohydrate removal, modification, or the like may be further added as needed.

### 3.2 Production of recombinant protein

Using a microorganism such as *Escherichia coli,* animal cells, or a plant, all or part of the profilin gene of *Toxoplasma gondii* may be introduced and expressed to prepare a recombinant protein.

A vector for preparing the recombinant protein can be obtained by linking (inserting) a gene encoding profilin into a known vector.

The gene encoding profilin may be one encoding the above-described amino acid sequence of profilin, and is properly optimized depending on the host to be described later. SEQ ID NO: 1 shows the nucleotide sequence of profilin gene (mRNA, complete cds) deposited in GenBank under Accession Number AY897579 (*Toxoplasma gondii* inflammatory profilin mRNA, complete cds), Version AY897579.1 GI:61612091. A gene may also be used which has about 60% or more, preferably about 70% or more, more preferably about 80% or more, particularly preferably about 90% or more, more particularly preferably about 95% or more, homology (identity) to SEQ ID NO: 1, provided that it encodes profilin (an variant or derivative thereof) having a desired immunogenicity.

The vector is not particularly limited provided that it can be replicated in a host; examples thereof include plasmid DNA and phage DNA. Examples of the plasmid DNA include *Escherichia coli*-derived plasmids (e.g., pBR322, pBR325, pUC18, pUC119, pTrcHis, and pBlueBacHis), *Bacillus* subtilis-derived plasmids (e.g., pUB110 and pTP5), yeast-derived plasmids (e.g., YEp13, YEp24, YCp50, and pYE52), and plasmids for plant cell hosts (pBI221, pBI121). Examples of the phage DNA include λ phage. In addition, an animal virus such as retrovirus or vaccinia virus and an insect virus vector such as baculovirus may be used.

To insert the gene of the present invention into a vector, for example, a method is adopted which involves first cutting purified DNA using a suitable restriction enzyme and inserting the resultant into the restriction enzyme site or multi-cloning site of a suitable vector DNA for linkage to the vector. The gene of the present invention needs to be introduced after operably linking the gene to a promoter depending on a host. Here, "operably" refers to that through the promoter activity, the gene of the present invention located downstream of the promoter is suitably expressed in a host to exert the function thereof. The type of the promoter used is properly determined depending on the host cell; however, the details will be described in the next section.

In addition to the promoter and the gene of the present invention, the vector of the present invention may contain, if desired, a cis element such as an enhancer, a splicing signal, a poly(A) addition signal, a transformation marker gene (for example, dehydrofolate reductase gene, ampicillin resistance gene, neomycin resistance gene, kanamycin resistance gene, hygromycin resistance gene, bialaphos resistance gene, carboxin resistance gene, and phleomycin resistance gene), a ribosome-binding sequence (SD sequence), and the like.

A transformant for producing a recombinant protein can be obtained by introducing the above vector into a suitable host. The host is not particularly limited provided that it can express the profilin gene of the present invention. Examples thereof include bacteria belonging to the genus *Escherichia* such as *Escherichia coli,* the genus *Bacillus* such as Bacillus subtilis, the genus *Pseudomonas* such as *Pseudomonas putida* and the genus *Rhizobium* such as *Rhizobium meliloti;* yeasts such as Saccharomyces cerevisiae and *Schizosaccharomyces pombe; Aspergillus oryzae;* animal cells such as COS cells and CHO cells; and insect cells such as Sf9 and Sf21.

When a bacterium such as Escherichia coli is used as a host, it is preferable that the vector of the present invention is capable of autonomous replication in the bacterium while comprising a promoter, a ribosome-binding sequence, the gene of the present invention, and a transcription termination sequence. The vector may also contain a gene controlling the promoter. Examples of the *Escherichia coli* include *Escherichia coli* HMS174 (DE3), K12 and DH1; examples of the *Bacillus subtilis* include *Bacillus subtilis* MI 114 and 207-21. The promoter therefor is not particularly limited provided that it enables expression in the above host such as *Escherichia coli;* examples thereof include promoters derived from Escherichia coli and phage, such as a trp promoter, a lac promoter, a PL promoter, and a PR promoter. Artificially designed/altered promoters such as a tac promoter may also be used. A method for introducing the vector into bacteria is not particularly limited; examples thereof can include a method using calcium ion [Cohen, S.N. et al.: Proc. Natl. Acad. Sci., USA, 69: 2110-2114 (1972)] and an electroporation method.

When yeast is used as a host, for example, *Saccharomyces cerevisiae, Schizosaccharomyces pombe,* or *Pichia pastoris* is used. The promoter therefor is not particularly limited provided that it enables expression in yeast; examples thereof can include a gal1 promoter, gal10 promoter, a heat shock protein promoter, an MFα1 promoter, a PH05 promoter, a PGK promoter, a GAP promoter, a ADH promoter, and a AOX1 promoter. A method for introducing the vector into the yeast is not particularly limited; examples thereof can include an electroporation method [Becker, D.M. et al.: Methods. Enzymol., 194: 182-187 (1990)], a spheroplast method [Hinnen, A. et al.: Proc. Natl. Acad. Sci., USA, 75: 1929-1933 (1978)], and a lithium acetate method [Itoh, H.: J. Bacteriol., 153: 163-168 (1983)].

When *Aspergillus oryzae* is used as a host, examples of the promoter include a GIaA promoter (Hata et al. Curr. Genet., Vol 22, 85-91, 1992), an AmyB promoter (Tuchiya et al. Biosci. Biotechnol. Biochem., Vol 46, 1849-1853, 1992), and a No. 8 promoter (Ozeki et al. Biosci. Biotech. Biochem., Vol 60, 383-389, 1996). A method for introducing the vector into *Aspergillus oryzae* is not particularly limited; for example, an electroporation method or a calcium ion method can be used.

The profilin protein can be obtained by culturing the above transformant (host cells) in a suitable medium and collecting a desired protein from the culture. The transformant may be cultured according to an ordinary method. For example, a transformant using a microorganism such as *Escherichia coli* or yeast as a host may be cultured in a natural or synthetic medium containing carbon and nitrogen sources and inorganic salts capable of being assimilated by the microorganism and enabling the transformant to be efficiently cultured. When a plant cell is used as a host, the transformant may be cultured in a medium for plant cells, to which vitamins such as thiamine and pyridoxine are added.

The carbon source used is a carbohydrate such as glucose, fructose, sucrose or starch, an organic acid such as acetic acid or propionic acid, or an alcohol such as ethanol or propanol. The nitrogen source used is ammonia, an inorganic or organic acid ammonium salt such as ammonium chloride, ammonium sulfate, ammonium acetate or ammonium phosphate, or any of other nitrogen-containing compounds, or peptone, meat extract corn steep liquor, or the like. The inorganic substance used is potassium primary phosphate, potassium secondary phosphate, magnesium phosphate, magnesium sulfate, sodium chloride, ferrous sulfate, manganese sulfate, copper sulfate, calcium carbonate, or the like.

In the medium, an antibiotic such as ampicillin or tetracycline may be added to the medium, if necessary. When a microorganism transformed with a vector using an inducible promoter as a promoter is cultured, an inducer may be added to the medium, if necessary. For example, isopropyl-β-D-thiogalactopyranoside (IPTG) or the like may be added to the medium when a microorganism transformed with a vector using a Lac promoter is cultured, and indoleacrylic acid (IAA) or the like, when a microorganism transformed with a vector using a trp promoter is cultured.

The culture is typically carried out at about 30 to 37°C for the order of 6 hours to 3 days under aerobic conditions such as shake culture and aerated and agitated culture. The pH is kept at a value of the order of 7.0 to 7.5 during the period of culture. The pH is adjusted using an inorganic or organic acid, an alkali solution, or the like. After culture, when the protein of the present invention is produced in bacterial bodies or in cells, the bacterial bodies or the cells are crushed to extract the protein. When the protein of the present invention is produced outside the bacterial bodies or outside the cells, the culture solution is directly used or subjected to removal of the bacterial bodies or the cells by centrifugation or the like. Thereafter, the protein of the present invention can be isolated and purified from the above culture using a general biochemical method used for the isolation and purification of protein, such as ammonium sulphate precipitation, SDS-PAGE, gel chromatography, ion exchange chromatography, hydrophobic chromatography or affinity chromatography, alone or in a proper combination thereof.

In addition to the above method, a peptide fragment containing a partial sequence or T-cell epitope of profilin may be prepared by protein engineering or by peptide synthesis.

### 4. Liposome

### 4.1 Lipid Constituting Liposome

The lipid constituting the liposome used for the present invention may be a common lipid known to constitute a liposome. Examples thereof include lipids derived from egg yolk, soybean, or other natural products from animals and plants, lipids whose unsaturation degree is reduced by hydrogenation thereof, or synthesized lipids; these lipids may be used alone or in a combination of a plurality thereof.

As will hereinafter be described, an amino group on a phospholipid is reacted with an aldehyde group of an oligosaccharide in the introduction of the oligosaccharide. Thus, the phospholipids used for the present invention are preferably those with amino groups, which may be used alone or in a combination of two or more thereof. The two fatty acid residues in the 1- and 2-positions of each phospholipid may be arbitrarily selected and may be those derived from natural products or synthetic products; fatty acid residues each having 4 to 30 carbons may be used which are derived from mixed fatty acids, saturated fatty acids, unsaturated fatty acids, polymerizable fatty acids, or the like. Preferred saturated fatty acids include those each having 12 to 24 carbons; examples thereof include lauric acid, myristic acid, palmitic acid, and stearic acid. Preferred unsaturated fatty acids include those each having 14 to 22 carbons and 1 to 6 unsaturated bonds; examples thereof include oleic acid, linoleic acid, and arachidonic acid. Of these phospholipids having amino groups, those derived from natural products are preferably phospholipids derived from egg yolk or soybean. Suitable examples thereof include phosphatidylethanolamine, phosphatidylserine or phosphatidylthreonine, dipalmitoylphosphatidylethanolamine, and dipalmitoyl phosphatidylcholine.

The membrane constituent (membrane-forming component) of the liposome may use a different compound capable of forming a liposome in addition to the phospholipid having an amino group, provided that a carbohydrate can be introduced thereinto. For example, compounds each conventionally used as a membrane constituent of a liposome may be used such as soybean lecithin, egg-yolk lecithin, phosphatidylglycerol, other phospholipids, cholesterols, fatty acids, and fatty acid salts.

Specific examples thereof include sterols such as cholesterol (Chol), 3β-[N-(dimethylaminoethane)carbamoyl]cholesterol (DC-Chol), and N-(trimethylammonioethyl)carbamoylcholesterol (TC-Chol); phosphatidylethanolamines such as dipalmitoylphosphatidylethanolamine (DPPE) and distearoylphosphatidylethanolamine (DSPE); phosphatidylcholines such as dipalmitoylphosphatidylcholine (DPPC) and distearoylphosphatidylcholine (DSPC); phosphatidylserines such as dipalmitoylphosphatidylserine (DPPS) and distearoylphosphatidylserine (DSPS); and phosphatidic acids such as dipalmitoylphosphatidic acid (DPPA) and distearoylphosphatidic acid (DSPA).

The liposome may be a multilamellar liposome or a unilamellar liposome. The particle diameter of the liposome used for the present invention is not particularly limited; however, it is 0.1 to 3 µm, preferably 0.2 to 2.5 µm. This is because a liposome particle diameter of more than the upper limit causes the liposomes to gelate, which does not enable the use thereof in a vaccine. The particle diameter of the liposome can be adjusted according to an ordinary method depending on the dosage form used, for example, by filtration using a filter having a desired pore size.

### 4.2 Oligosaccharide on Surface of Liposome

The liposome used for the present invention has an oligosaccharide capable of binding to a carbohydrate recognition molecule on the surface of antigen-presenting cells on the surface of the liposome. Here, the "antigen-presenting cells" mean macrophages, dendritic cells, or the like. On the surface of antigen-presenting cells are present an Fc receptor and a complement receptor, a scavenger receptor, a mannose receptor, a lipopolysaccharide (LPS) receptor, CD11b/CD18 (CR3) also as a complement receptor, a Toll-like receptor, and the like, which directly or indirectly play important roles in the phagocytosis of bacteria and the like via a carbohydrate and in the uptake of a glycoprotein in an exogenous foreign material and the presentation of its antigen. The "carbohydrate recognition molecule on the surface of antigen-presenting cells" according to the present invention means any molecule having carbohydrate-binding lectin-like properties present on the surface of antigen-presenting cells as described above; preferred examples thereof can include a mannose receptor.

The oligosaccharide on the surface of the liposome is not particularly limited provided that it can bind to the above-described carbohydrate recognition molecule on the surface of antigen-presenting cells; examples of sugar residues constituting the oligosaccharide include D-mannose (D-Man), L-fucose (L-Fuc), D-acetylglucosamine (D-GlcNAc), D-glucose (D-Glc), D-galactose (D-Gal), D-acetylgalactosamine (D-GalNAc), and D-rhamnose (D-Rha). The oligosaccharide is preferably a high-mannose type oligosaccharide consisting of sugar residues including D-mannose; among others, preferred are an oligosaccharide consisting of D-mannose and an oligosaccharide consisting of D-mannose and D-acetylglucosamine, and particularly, an oligosaccharide consisting only of D-mannose is most preferable. Examples of the oligosaccharide consisting of D-mannose can include mannobiose (Man2), mannotriose (Man3), mannotetraose (Man4), mannopentaose (Man5), mannohexaose (Man6), and mannoheptaose (Man7).

The binding between the sugar residues constituting the oligosaccharide is not particularly limited; examples thereof can include α1→2 binding, α1→3 binding, α1→4 binding, α1→6 binding, and β1→4 binging. The sugar residues may bind one after another in a straight chain form or may bind so as to form a branched structure.

The number of the sugar residues constituting the oligosaccharide is preferably 2 to 11, particularly 3 to 11, and most preferably about 3 to 5.

The amount of the oligosaccharide based on the liposome varies depending on the type of the oligosaccharide, the type of a *Toxoplasma gondii-derived* soluble protein to be included, the combinational structure of the liposome, and the like; however, it is typically 0.5 µg to 500 µg based on 1 mg of the lipid constituting the liposome.

The oligosaccharide can be introduced into the liposomes using an artificial glycolipid prepared by causing the oligosaccharide to bind to a lipid. The artificial glycolipid can be prepared by causing the oligosaccharide to bind to the lipid by reacting an aldehyde group of the oligosaccharide with a phospholipid having an amino group to form a Schiff base and then reducing the Schiff base according to an ordinary method, preferably by chemical reduction, for example, by reduction using NaBH₃CN (Tsugio Mizuochi, Toshitsu Kogaku (in Japanese), 224-232, 1992). Subsequently, using the artificial glycolipid, the oligosaccharide is introduced into the liposomes. When the artificial glycolipid is water-soluble and not sufficiently dissolved in an organic solvent (for example, when the artificial glycolipid uses a bound substance of the above-described RN and DPPE (RN-DPPE)), an aqueous solution thereof (of RN-DPPE) is prepared and mixed with the liposomes, which is then incubated, for example, at 4°C to 80°C (preferably, a temperature at which an inclusion is not denatured), room temperature or a phase transition temperature for 0.5 to 120 hours, for example, for about 24 hours. When the artificial glycolipid is soluble in an organic solvent, the artificial glycolipid may be dissolved together with a lipid for constituting a liposome in the organic solvent during a liposome production process to form liposomes according to an ordinary method. In this respect, the binding of the oligosaccharide to the surface of the liposome may be examined by whether a liposome aggregation reaction occurs by the addition of a carbohydrate recognition molecule or a portion thereof present on the surface of the antigen-presenting cells.

### 4.3 Making Liposome formulation

The liposome formulation of the present invention is produced by including the above-described profilin protein in the liposomes. The amount of the profilin protein included is not particularly limited and can be properly adjusted depending on the administration route thereof; however, it is preferably typically 0.1 µg to 500 µg based on 1 mg of the lipid used in the liposome.

### 5. Vaccine Formulation

The vaccine formulation of the present invention is prepared such that it can be administered in the form of a solution or a suspension by properly adding a pharmaceutically acceptable carrier to the liposome formulation of the present invention. A pharmaceutically acceptable diluent compatible with the active ingredient is often mixed in the vaccine formulation of the present invention. Suitable diluents include, for example, water, saline, dextrose, glycerol, ethanol, and a mixture thereof. In addition, the vaccine may contain a small amount of an auxiliary substance (for example, a humidifying agent or an emulsifying agent), a pH buffering agent, and an adjuvant for enhancing the efficacy of the vaccine, if desired. Examples of adjuvants which can be efficacious include, but not limited to, the following: aluminium hydroxide, N-acetyl-muramyl-L-threonyl-D-isoglutamine (thr-MDP), N-acetyl-nor-muramyl-L-alanyl-D-isoglutamine (called CGP11637, nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (called CGP19835A, MTP-PE), and RIBI. RIBI contains three ingredients extracted from bacteria, i.e., monophosphoryl lipid A, trehalose dimycolate and cell wall skeleton (HPL+TDM+CWS), in a 2% squalene/Tween^{®} 80 emulsion. The efficacy of the adjuvant can be determined by measuring the amount of an antibody generated after administering a vaccine comprising profilin.

The vaccine formulation of the present invention is typically administered by injection such as subcutaneous, intravenous or intramuscular injection. Different prescriptions suitable for other administration modes include a suppository and in some cases oral, ophthalmic, enteral and nasal prescriptions.

If desired, one or more compounds having an adjuvant activity may be added. Adjuvants are non-specific stimulatory factors of the immune system. They enhance the immune response of a host to the vaccine. Specific examples of adjuvants known in the art include Freund's complete and incomplete adjuvants, vitamin E, non-ionic block polymer, muramyldipeptide, saponin, mineral oil, vegetable oil, and carbopol. Examples of adjuvants suitable particularly for application to the mucous membrane include *Escherichia coli* (*E. coli*) heat-labile toxin (LT) or cholera toxin (CT). Other suitable adjuvants include, for example, aluminum hydroxide, ammonium phosphate or aluminium oxide, oil emulsion (e.g., Bayol(Trademark) or Marcol 52(Trademark)), saponin or vitamin E solubilisate. Thus, a preferred form of the vaccine of the present invention contains an adjuvant.

For example, in an injectable solution administered subcutaneously, intradermally, intramuscularly or intravenously, specific examples of a different pharmaceutically acceptable carrier or diluent capable of administered together with the vaccine of the present invention include a stabilizer, a carbohydrate (e.g., sorbitol, mannitol, starch, cane sugar, glucose, or dextran), a protein such as albumin or casein, a protein-containing material such as bovine serum or skim milk, and a buffer (e.g., phosphate buffer).

The amount to be administered, of the antigen typically ranges from 0.01 µg to 100,000 µg per administration and depends on the subject treated (e.g., mammal or birds such as humans, pigs, sheep, goats, deer, or cats), the ability of the subject to synthesize antibody in the immune system thereof and the desired degree of protection and also depends on the administration route such as the oral, subcutaneous, nasal, ophthalmic, enteral, intradermal, intramuscular or intravenous route of administration.

The vaccine formulation of the present invention can be given on a single administration schedule or preferably on a multiple administration schedule. For the multiple administration schedule, individual administration can be carried out 1 to 10 times in a beginning period of vaccination, followed by performing another administration at a time interval necessary for maintaining or boosting the immune response, for example, after 1 to 4 months as the second administration. Subsequently, administration can be carried out after several months, if necessary. An administration regimen is also at least partially determined by the individual's necessity and depends on the doctor's judgment.

In addition, the vaccine of the present invention may be prophylactically used for fresh *Toxoplasma gondii* infection. Further, it may be used as a therapeutic vaccine for eliminating *Toxoplasma gondii* by the administration thereof to a subject infected with *Toxoplasma gondii* for the induction of a strong immune reaction to *Toxoplasma gondii* in the living body.

The vaccine of the present invention may be preventively or therapeutically used against a different pathogen having an amino acid sequence having 70% or more identity with profilin of *Toxoplasma gondii.*

### Examples

The present invention is described below in detail, based on Examples. However, the invention is not intended to be limited by these Examples.

### Example 1

### Preparation of Toxoplasma gondii-Derived Soluble Protein

Profilin, cyclophilin 18, dense granule protein 14 genes (GenBank ID AY897579, TGU04633 and FJ015061, respectively) were cloned from *Toxoplasma gondii.* Based on each of the genes, a recombinant protein in which glutathione S-transferase (GST) was fused was expressed in *E. coli.* The resultant recombinant protein was purified using Glutathione Sepharose 4B (from Amersham Pharmacia Biotech Inc.) and GST was removed with thrombin protease (from GE Healthcare Inc.). In addition, using Detoxi-Gel^{™} Endotoxin Removing Gel (from Pierce Inc.), endotoxin was removed from the purified recombinant protein.

### Example 2

### Preparation of Artificial Glycolipid

To 2.5 to 5 mg of mannotriose (Man3) having the structure: Manα1→6 (Manα1→3) Man was added 600 µl of distilled water, which was then stirred for dissolution to prepare an oligosaccharide solution. On the other hand, DPPE was dissolved to a concentration of 5 mg/ml in a chloroform/methanol (1:1 by volume ratio) mixed solution to prepare a DPPE solution. NaBH₃CN was dissolved to a concentration of 10 mg/ml in methanol to prepare a NaBH₃CN solution. To 600 µl of the oligosaccharide solution were added 9.4 ml of the DPPE solution and 1 ml of the NaBH₃CN solution, which was then stirred and mixed. The reaction mixture was incubated at 60°C for 16 hours to form an artificial glycolipid. The reaction mixture was purified using a silica gel column and a C18 reverse-phase column to provide an artificial glycolipid, M3-DPPE.

### Example 3

### Preparation of Profilin Protein-Including Liposome

Cholesterol, dipalmitoylphosphatidylcholine (DPPC) and mannotriosedipalmitoylphosphatidylethanolamine (M3-DPPE) prepared in Example 2 or cholesterol and dipalmitoylphosphatidylcholine (DPPC) were mixed at a molar ratio of 10:10:1 or 1:1, respectively and dissolved in 2 ml of chloroform to prepare a lipid film in a 10-ml pear-shaped flask. To the lipid film was subsequently added 3.75 mg/ml of the *Toxoplasma gondii*-derived soluble protein obtained in Example 1 (content: 0.5 mg/ml) to prepare liposomes by vortexing in a water bath at 40°C. The particle sizes of these liposomes were then selected 5 times while applying a pressure ranging from 0.2 to 1 MPa to a 1-µm filter using an extruder as a particle-size selector. Subsequently, the liposome solution was recovered by a centrifugation method and suspended using PBS (-) three times. The suspension was centrifuged, followed by removing the supernatant to remove the antigen not included in the liposomes. The resultant liposomes were analyzed to measure the cholesterol amount and the *Toxoplasma gondii-*derived soluble protein using commercially available kits, Cholesterol E Test Wako (from Wako Pure Chemical Industries Ltd., 439-17501) and Modified Lowry Protein Assay Reagent Kit (from Pierce, 23240).

### Example 4

### Verification of Vaccine Effect of Toxoplasma gondii-Derived Soluble Protein-Including Liposome

For abbreviations of the liposome inclusions used in Examples below, "GST-OML" indicates a liposome having an oligosaccharide (Man3) on the surface, in which GST protein prepared as in Example 1 is included; "TgPF-OML" indicates a liposome having an oligosaccharide (Man3) on the surface, in which profilin protein prepared as in Example 1 is included; "TgCyp-OML" indicates a liposome having an oligosaccharide (Man3) on the surface, in which cyclophilin 18 protein prepared as in Example 1 is included; and "TgGRA14-OML" indicates a liposome having an oligosaccharide (Man3) on the surface, in which dense granule protein 14 protein prepared as in Example 1 is included. "PBS" indicates a phosphate buffer solution.

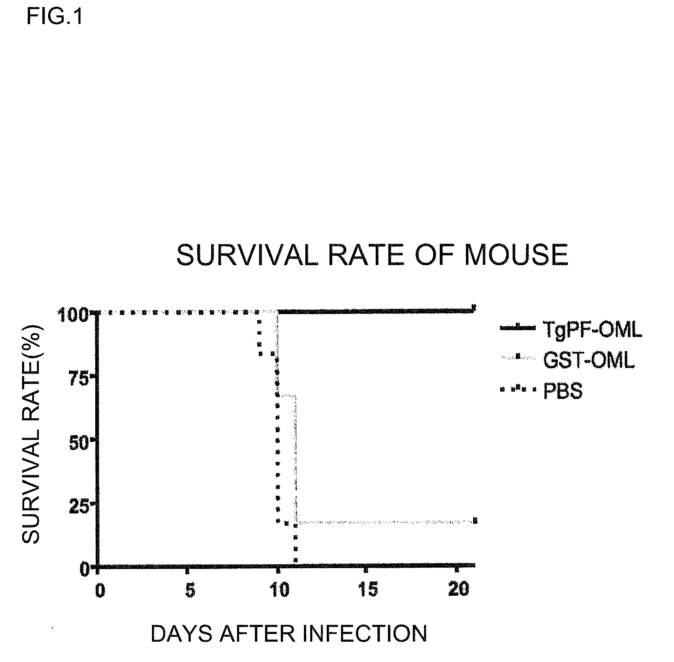

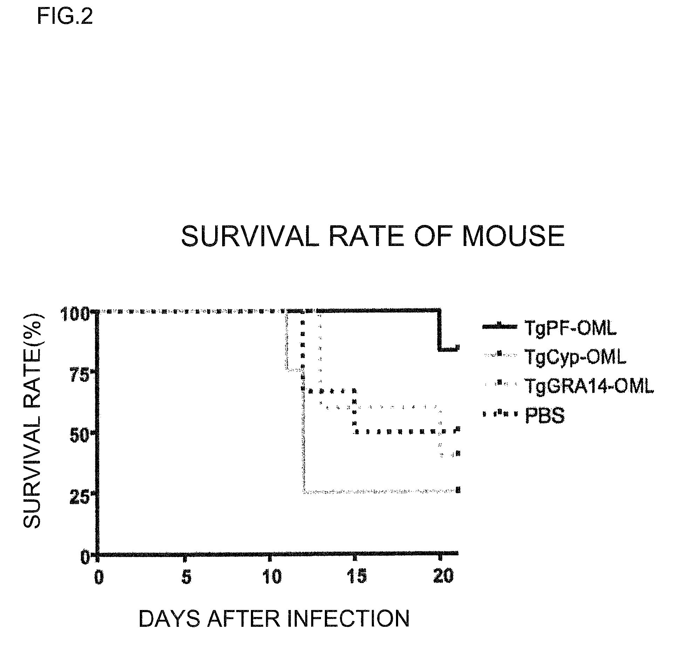

C57BL/6J mice (8 weeks old, female) were subcutaneously inoculated with each of *Toxoplasma gondii-*derived soluble protein-including liposomes (40 pmol in terms of protein) and PBS three times at 2-week intervals. Two weeks after the 3rd inoculation, the mice were intraperitoneally inoculated with 1,000 cells of *Toxoplasma gondii* (tachyzoite). The survival rate of the mice was measured during 21 days after infection. In a first experiment, a TgPF-OML-inoculated group and a GST-OML-inoculated group were compared (Figure 1). In a second experiment, the TgPF-OML-inoculated group was compared with groups inoculated with OMLs including TgCyp and TgGRA14 as other *Toxoplasma gondii* soluble proteins (Figure 2).

When the 21-day survival rate was compared between the experimental groups of mice, a high survival rate was observed in the TgPF-OML-inoculated group. For the inoculation with OML including each of TgCyp and TgGRA14 as the other *Toxoplasma gondii* soluble proteins, no increase in the survival rate was observed when compared to the group inoculated with PBS as control.

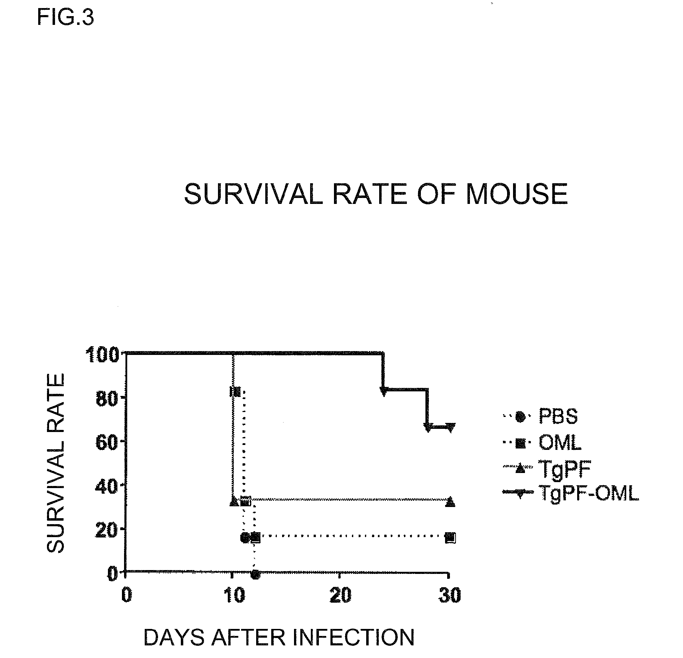

To verify the effect of antigen inclusion in OML in the protective effect against infection, C57BL/6J mice (8 weeks old, female) were subcutaneously inoculated with each of TgPF-including liposomes (40 pmol in terms of protein), OML alone, 40 pmol of TgPF, and PBS three times at 2-week intervals. Two weeks after the 3rd inoculation, the mice were intraperitoneally inoculated with 1,000 cells of *Toxoplasma gondii* (tachyzoite). The survival rate of the mice was measured during 30 days after infection (Figure 3). As a result, whereas a high survival rate was observed in the TgPF-OML-inoculated group, no protective effect against infection was observed in the TgPF alone-inoculated group.

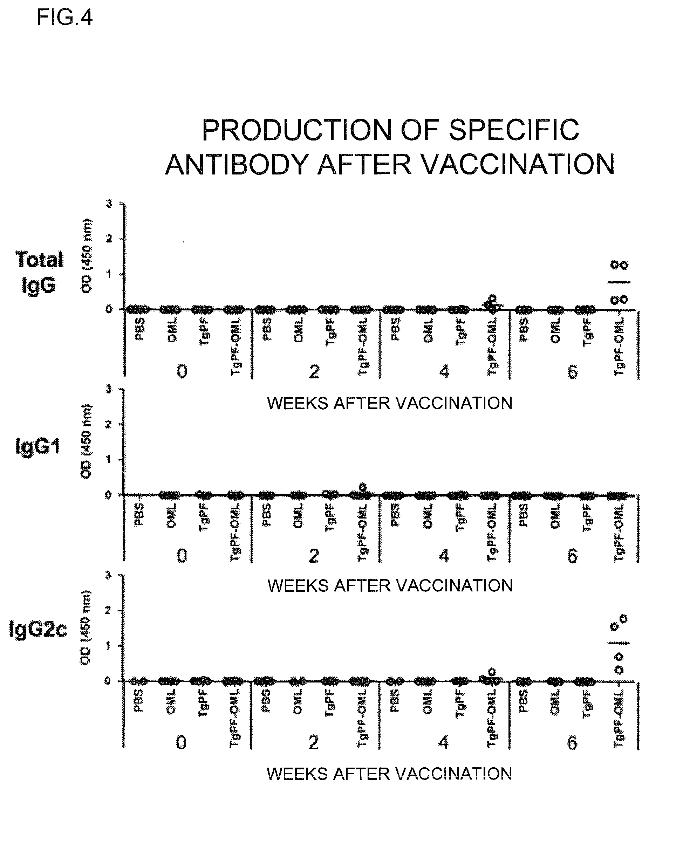

C57BL/6J mice (8 weeks old, female) were subcutaneously inoculated with each of TgPF-including liposomes (40 pmol in terms of protein), OML alone, 40 pmol of TgPF, and PBS three times at 2-week intervals. Blood was collected before the first administration and at the 2nd, 4th, and 6th week after the first administration to provide plasma. Antigen-specific IgG, IgG1, and IgG2c antibodies were measured by an ELISA method with TgPF as an antigen (Figure 4).

In the TgPF-OML-inoculated group, the production of TgPF-specific IgG and IgG2 antibodies were observed at the 6th week after priming. No TgPF-specific antibody was produced in experimental groups other than the TgPF-OML-inoculated group. These results showed that TgPF could be immunized by inclusion in liposomes to promote the production of TgPF-specific antibodies.

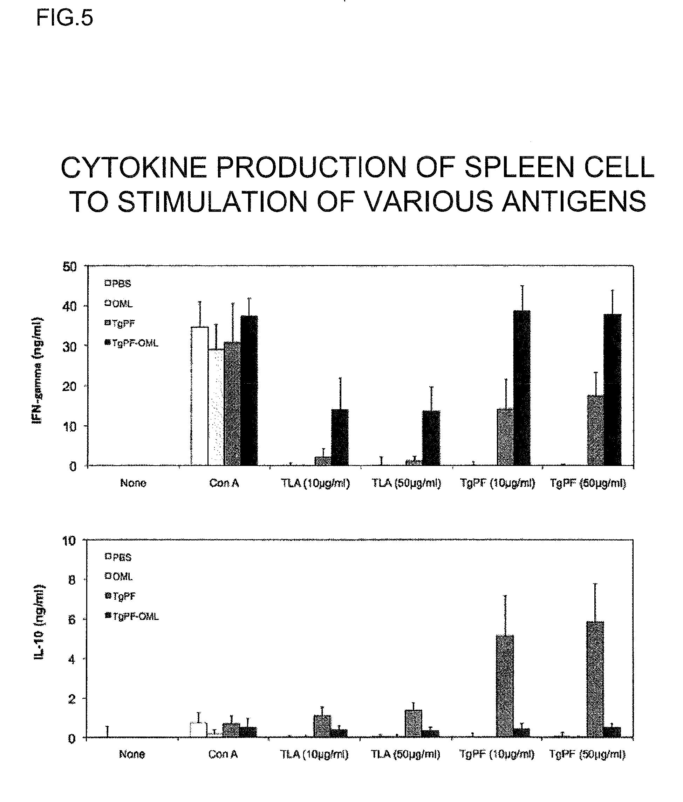

C57BL/6J mice (8 weeks old, female) were subcutaneously inoculated with each of TgPF-including liposomes (40 pmol in terms of protein), OML alone, 40 pmol of TgPF, and PBS three times at 2-week intervals. At the 2nd week after the final administration, spleen was removed and homogenated to prepare a spleen cell suspension (5 × 10⁶ cells/ml, RPMI1640 medium). The spleen cell suspension from each individual was cultured in a CO₂ incubator for 48 hours in the presence of the *Toxoplasma gondii* soluble antigen (TLA, final concentration: 10 µg/ml or 50 µg/ml), TgPF (final concentration: 10 µg/ml or 50 µg/ml), or concanavalin A (final concentration: 0.5 µg/ml) or under unstimulated conditions (none), followed by recovering the culture supernatant. Interferon gamma (IFN-γ, Th1 was used as a reaction index) and interleukin (IL)-10 (IL-10, Th2 was used as a reaction index) in the recovered culture supernatant were measured by an EIA method (Figure 5).

As a result of measuring the amount of IFN-γ production in spleen cells, the spleen cells of the TgPF-OML-inoculated group were found to produce IFN-γ in response to the stimulation by TLA and TgPF. This production was at a higher level than that for the IFN-γ production of the spleen cells in the TgPF alone-inoculated group. No production of IFN-γ from spleen cells to antigen stimulation was observed in the PBS-inoculated group and the OML-inoculated group.

As a result of measuring IL-10 production in spleen cells, the spleen cells of the TgPF alone-inoculated group were found to produce IL-10 in response to the stimulation by TLA and TgPF. The production was at a higher level than that for IL-10 production of the spleen cells in the TgPF-OML-inoculated group. No production of IFN-γ from spleen cells to antigen stimulation was observed in the PBS-inoculated group and the OML-inoculated group.

These results demonstrated that oligosaccharide liposomes in which profilin as a *Toxoplasma gondii* soluble protein is included can be subcutaneously administered to induce antigen-specific Th1 immunity for protection from *Toxoplasma gondii* infection. Thus, the oligosaccharide liposome in which the profilin protein was included according to the present invention could be used as an effective vaccine for *Toxoplasma gondii* infection.

### Industrial Applicability

According to the present invention, a vaccine formulation for *Toxoplasma gondii* infection is provided which is safe and has a high prophylactic effect. The vaccine formulation according to the present invention is useful in humans and the livestock industry and the like in which *Toxoplasma gondii* infection is serious.

All publications, patents, and patent applications cited in this application are intended to be incorporated herein by reference in their entirety.

## Claims

1. A liposome formulation in which *Toxoplasma gondii-*derived profilin or an immunologically active variant or derivative thereof, or an immunologically active fragment thereof is included in liposomes each having an oligosaccharide capable of binding to a carbohydrate recognition molecule on the surface of antigen-presenting cells on the surface of the liposome.

2. The liposome formulation according to claim 1, wherein the profilin or an immunologically active variant or derivative thereof is any of the following (a) to (c):
(a) a protein comprising the amino acid sequence shown in SEQ ID NO: 2,
(b) a protein comprising an amino acid sequence in which one or several amino acids are deleted, substituted, or added in the amino acid sequence shown in SEQ ID NO: 2 and capable of inducing an immune response to *Toxoplasma gondii,*
(c) a protein comprising an amino acid sequence having 80% or more identity with the amino acid sequence shown in SEQ ID NO: 2 and capable of inducing an immune response to *Toxoplasma gondii.*

3. The liposome formulation according to claims 1 or 2, wherein the carbohydrate recognition molecule on the surface of antigen-presenting cells is a mannose receptor.

4. The liposome formulation according to any one of claims 1 to 3, wherein the oligosaccharide consists of 2 to 11 sugar residues.

5. The liposome formulation according to any one of claims 1 to 4, wherein the oligosaccharide consists of 3 to 5 sugar residues.

6. The liposome formulation according to any one of claims 1 to 5, wherein the oligosaccharide comprises two or more mannose sugar residues.

7. A vaccine formulation for *Toxoplasma gondii* infection, comprising the liposome formulation according to any one of claims 1 to 6 and a pharmaceutically acceptable carrier.

8. The vaccine formulation according to claim 7, wherein the vaccine formulation is subcutaneously, intradermally, orally, intraperitoneally, or nasally administered.
